# EUROPEAN PATENT SPECIFICATION

(11) **EP 2 448 827 B1**
(45) Date of publication and mention of the grant of the patent: **18.06.2014**
(21) Application number: 10794441.5
(22) Date of filing: 24.06.2010
(51) Int. Cl.: A61L 2/20, B65B 31/02, B65B 31/04, B65B 55/02, B65B 55/10, F24F 13/28, F24F 3/16

(54) **DEVICE FOR CLEANED AIR PROVISION**
VORRICHTUNG ZUR BEREITSTELLUNG GEREINIGTER LUFT
DISPOSITIF POUR FOURNITURE D'AIR NETTOYE

(30) Priority: 03.07.2009 SE 0900908
(43) Date of publication of application: 09.05.2012
(73) Proprietor: Tetra Laval Holdings & Finance S.A., 1009 Pully (CH)
(72) Inventor: LINDBLAD, Ulf, 224 56 Lund (SE); OLSSON, Jenny, 237 33 Bjärred (SE)
(74) Representative: Tetra Pak - Patent Attorneys SE
(86) International application number: PCT/SE2010/000180
(87) International publication number: WO 2011/002383

(56) References cited:
- EP-A2- 1 645 813
- DE-A1- 19 945 500
- JP-A- 3 103 258
- US-A- 3 513 627
- US-A- 6 105 634

## Description

### Technical Field

The present invention relates to a device for providing a flow of cleaned air to an apparatus. The device comprises a bent duct having a first end arranged to be positioned outside the apparatus and a second end arranged to be positioned inside the apparatus, and a filter. Air from outside of the apparatus is conveyed through the filter for cleaning, through the duct and into the apparatus.

### Technical Background

Within the food industry, beverages and other products are often packed in paper or paperboard based packages. Packages intended for liquid food are often produced from a packaging laminate comprising a core layer of paper or paperboard and an outer, liquid-tight layer of thermoplastic material on at least that side of the core layer which will form the inside of the packages.

One kind of frequently occurring packages are the so-called carton bottles. In substance, these are composed of a lower part in the form of a sleeve of packaging laminate like the one described above, and an upper part in the form of a plastic top having a neck which is provided with an opening/closing means, such as a screw cap.

In a known packaging machine, carton bottles which are open in the bottom, i.e. the sleeve end, are produced. Then, the open carton bottles are transported, disposed upside-down on a conveyor belt, to a sterilization station for at least inside sterilization in order to extend the shelf-life of the product to be packed in the carton bottles. Depending upon the desired length of shelf-life, and depending upon whether the carton bottles are to be distributed and stored in a refrigerated environment or at room temperature, different levels of sterilization may be selected. After sterilization the carton bottles are further transported to a filling station for product filling, a sealing station for sealing of the open bottom and a final folding station for final folding of the bottom. The sterilization, filling, sealing and final folding stations are comprised in an apparatus, in turn, comprised in the packaging machine.

In order not to risk recontamination of the inside of the carton bottles, it is important to maintain an aseptic environment in at least an upper part of the sterilization, filling and sealing stations of the apparatus, which upper part is arranged to accommodate the open bottoms of the carton bottles conveyed through the apparatus. In the known packaging machine, as one action for maintaining the desired aseptics, sterile air is continuously introduced at the top of the apparatus in the area of the sealing station. The sterile air is provided through a duct also comprised in the packaging machine, a first end of the duct being arranged outside the apparatus and a second end of the duct being arranged inside the apparatus. The duct is provided with a sterile filter and air from the surroundings is filtered there through before being introduced in the apparatus. Most commercially available filters have a rectangular shape and for the filter to fit the duct properly, the duct has a corresponding rectangular cross section.

The sealing of the filled open carton bottles takes place at the second end of the duct. In connection with the carton bottles being squeezed and flat-laid in the bottom to effect the bottom sealing the filled product may splash. If the filter is hit by the splashes and thereby contaminated, the desired aseptics in the apparatus could be jeopardized. The filters used in this type of application are not washable. Therefore, to protect the filter from splashing, the duct is bent approximately 90 degrees and the filter is arranged before the bend seen in a direction from the first (outside) end towards the second (inside) end of the duct. Any product splashes will thereby hit the inside walls of the duct and not the filter since this is hidden beyond the bend. The inside walls of the duct can relatively easy be cleaned which makes it easier to obtain the desired aseptics in the apparatus.

However, inside a bent duct of the above described type, a separation of the flow through the duct will arise at the bend. Such flow separation may result in that air flows in undesired directions are established inside the duct. This involves a risk of that air from inside of the apparatus, possibly from parts of the apparatus where aseptic conditions do not prevail, being sucked into the duct which could jeopardize the desired aseptics inside the apparatus.

Attempts have been made to solve the above issue by, instead of using a bent duct, arranging perforated plates inside a duct being straight, below the filter. The purpose of the perforated plates is to protect the filter by collecting any product splashes resulting from the carton bottle bottom sealing. However, these perforated plates are relatively hard to clean which makes it hard to obtain the aseptic conditions at the sealing station. Further, there is a risk of product splashed on the plates dripping from the same, down into the not yet sealed packages.

### Summary

An object of the present invention is to provide a device for providing a flow of cleaned air to an apparatus which, at least partly, eliminate potential limitations of prior art. The basic concept of the invention is to provide the duct with a bend for protecting the filter and a varying cross section for preventing flow separation inside the duct to enable maintenance of desired conditions inside the apparatus.

The device for achieving the object above is defined in the appended claims and discussed below.

A device for providing a flow of cleaned air to an apparatus according to the invention comprises a bent duct having a first end arranged to be positioned outside the apparatus and a second end arranged to be positioned inside the apparatus, and a filter. Air from outside of the apparatus is conveyed through the filter for cleaning, through the duct and into the apparatus. The device is inter alia characterized in that a polygonal cross section of the duct orthogonal to a longitudinal centre axis of the same varies, the cross section at the first end differing from the cross section at the second end.

The air to be provided to the apparatus can have different degrees of purity depending on the specific application. As an example, the air to be provided to the apparatus could be sterile, in which case a sterile filter, such as a HEPA filter, could be used for filtering the air.

The duct can be bent in different ways, e.g. it can be bent approximately 90 degrees or be more smoothly curved or even twisted, one or more times. Because of the bend, the filter can be protected from splashing as described above. Of course, for this to be possible, the filter must be arranged at the right side of the bend, i.e. upstream of the bend seen in the desired flow direction in the duct.

As mentioned by way of introduction, commercially available filters have predetermined shapes, more particularly, they are rectangular, and in order for the filter to be easily and effectively used in connection with the duct, the duct cannot have any configuration, at least not in an area of cooperation with the filter. However, as explained above, if the duct has a throughout rectangular cross section and comprises a bend, flow separation will arise inside the duct. By varying the cross section of the duct in an appropriate way, flow separation can be prevented, or at least heavily reduced, which enables the establishment of an essentially one-way flow inside the duct and between the duct and the apparatus, in turn enabling maintenance of desired conditions inside the apparatus.

The cross section of the duct can be appropriately altered in a number of different ways. As an example, the inventive device can be so constructed that said cross section is gradually altered along the longitudinal centre axis of the duct, from the cross section at the first end to the cross section at the second end. In accordance therewith, the transition from the first to the second cross section occurs smoothly and successively, which minimizes the risk of flow disturbances arising in the duct.

The device according to the invention can be such that the filter is arranged to occupy essentially the entire cross section of the duct at a position of the filter. Such an embodiment enables a relatively mechanically simple construction of the inventive device since it enables use of a minimum of additional components for connecting the duct and the filter. The filter can be arranged in differrent positions in relation to the duct, both inside and outside the duct.

As previously mentioned, in a bent duct with a through rectangular cross section, there is a risk of flow separation arising inside the duct, at the bend, the sharper bend, the higher risk and the stronger flow separation. Further, inside a duct with a rectangular or triangular cross section, bent or not, also other flow disturbances, so-called corner effects, may arise. This means that secondary flows or circulation zones are formed at the corners of the duct, the sharper corners, the larger circulation zones. For this reason, the inventive device may be so constructed that the duct cross section at the second end has more (and thereby more blunt) corners than the duct cross section at the first end. In other words, according to this embodiment, the duct cross section at the second end should be "rounder" than the duct cross section at the first end to enable a reduction of the corner effects at the second end. It should be said that circles, ellipses and other round geometric forms herein are regarded as comprising an infinite number of corners.

In accordance with the above paragraph, according to an embodiment of the present invention, the cross section at the first end is rectangular and the cross section at the second end is octagonal. This embodiment is advantageous since rectangular filters are easily accessible on the market. Another advantage is that a particularly simple construction of the duct is enabled since a rectangle relatively easily can be transformed into an octagon by cutting off the four corners.

According to one embodiment of the present invention, the cross section of the duct at the first end is larger than the cross section of the duct at the second end. This feature is advantageous since it will assist in promoting a flow through the duct in the desired direction, i.e. from the first to the second end thereof.

The inventive device may comprise a duct which is bent in such a way that a direction of the longitudinal centre axis at the first end is angled in relation to a direction of the longitudinal centre axis at the second end. Such a design enables, as will be apparent from the following section "Detailed Decription", a mechanically simple and straightforward construction of the invention, simple cleaning of the duct and easy access to the filter.

According to one embodiment of the present invention, the duct extends a distance into the apparatus to partly enclose a zone where sealing of filled, open packages takes place. This means that the package sealing is, in fact, performed inside the duct. As was described by way of introduction, after sterilization at least a part of the packages should be kept in a certain environment until they are closed by bottom sealing to prevent contamination. By the duct partly enclosing the sealing zone and an essentially one-way flow of cleaned air being provided through the duct, the achievement of this object is facilitated.

In accordance with the above embodiment, the duct may further be arranged to extend into the apparatus through a top wall thereof, the duct being arranged separated from side and bottom walls of the apparatus to enable a free way for the flow of air out from the duct. This arrangement is advantageous since it eliminates, or at least heavily reduces, the risk of that air about to be fed out of the duct changes its flow direction and starts to circulate inside the duct. Such a disturbance of the desired one-way flow through the duct could jeopardize the desired conditions inside the apparatus.

The device could, in accordance with one embodiment of the invention, be such that the duct, at the second end, comprises two opposing openings extending along the longitudinal center axis thereof. The provision of these openings enables package passing through the duct in a direction perpendicular to the longitudinal center axis thereof, which is necessary if operations on the packages is to be performed inside the duct.

Naturally, the above characteristics may naturally be combined in the same embodiment.

### Brief Description of the Drawings

Fig. 1 is a schematic side view of part of a packaging machine comprising an apparatus and a device according to the present invention.
Fig. 2 schematically illustrates a cross section of the packaging machine of Fig. 1, taken along the line A-A.
Fig. 3 schematically illustrates a cross section of a part of the packaging machine of Fig. 1, taken along the line B-B.
Fig. 4 schematically illustrates how a cross section of a duct comprised in the device according to the invention is varying along a longitudinal center axis thereof.

### Detailed Description

In the following, the term (adequate or the like) sterile is taken to signify that the package, after sterilization, attains a level of sterilization which is designnated commercially sterile.

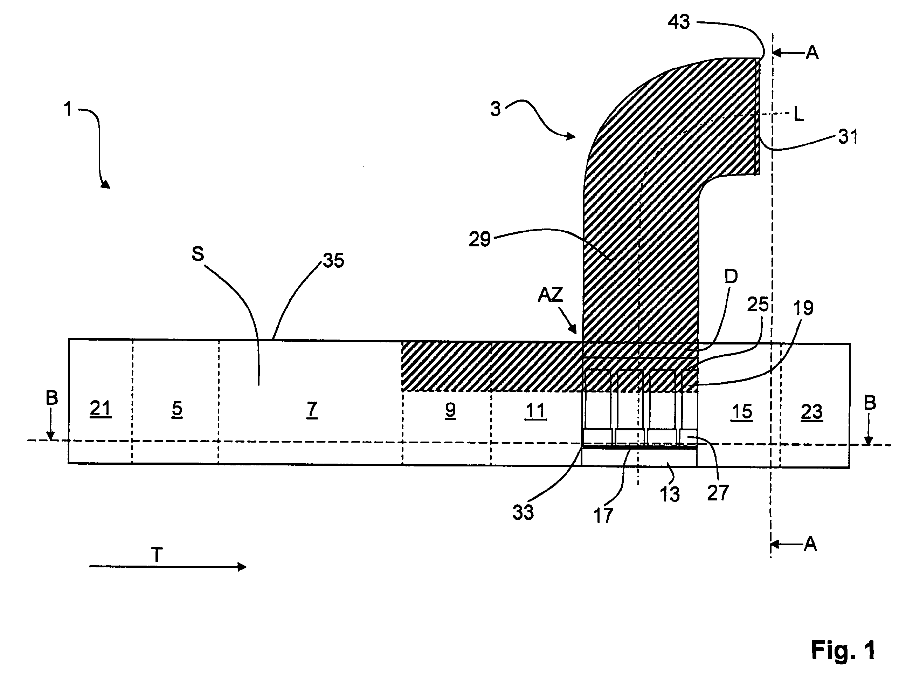

In figure 1, a packaging machine (not illustrated in its entirety) for producing filled, finished packages in the form of carton bottles of the initially described type is shown. The packaging machine comprises an apparatus 1 for sterilization, filling, sealing and folding of the carton bottles cooperating with a device 3 for provision of a flow of cleaned air to the apparatus, more particularly sterile air, according to one embodiment of the present invention, which device is also comprised in the packaging machine. For illustrative purposes, as apparent from the figure, the apparatus 1 has been opened up (a side wall S has been partly removed) in the area of interconnection with the inventive device 3. For the same reason, to enable looking into the device 3, it has been opened up by partial removal of a wall D in the area of interconnection with the apparatus 1.

The apparatus 1 is adapted for gas phase sterilization of the carton bottles prior to filling, sealing and folding of the same. To this end, the apparatus 1 comprises a preheating zone 5, a gassing zone 7 and a venting zone 9. The apparatus 1 further comprises a filling zone 11, a sealing zone 13, a final folding zone 15 and a conveyor 17 for transporting the carton bottles 19 through the various zones in a transport direction T. Further, the apparatus 1 has an infeed station 21 and an outfeed station 23 for the carton bottles 19. The boundaries between the zones, and the zones and the stations, have been illustrated with broken lines in the figures.

As indicated by the name, the carton bottles 19 are fed, with their bottom ends 25 open, to the apparatus 1 via the infeed station 21 where they are arranged upside-down, with their respective bottom end 25 directed upwards, in carrier means 27 attached to the conveyor 17. Arranged like this, the carton bottles are then moved through the zones 5, 7 and 9 for sterilization. In the gassing zone 7, the carton bottles 19 are exposed to gaseous hydrogen peroxide. In order to prevent the hydrogen peroxide from condensing on the surface of the carton bottles in the gassing zone 7, which impedes later removal, the carton bottles are heated up in the preheating zone 5 to a temperature above the dew point of the hydrogen peroxide gas. In the venting zone 9, the carton bottles are subjected to sterile hot air in order to vent off hydrogen peroxide which remains in and on the carton bottles. This sterilization operation is described in more detail in Swedish copending patent application filed by the applicant on the same date as the present application and titled "A device and a method for sterilization of packages" (SE-0900907-7), which application is hereby incorporated herein by reference. After sterilization, the carton bottles are fed to the filling zone 11 for filling of the desired product, the sealing zone 13 for bottom sealing and the final folding station 15 for final forming. In the sealing zone 13, the carton bottles 19 are squeezed, and thereby flat-laid, and then sealed along a transverse bottom sealing area for closing the carton bottles. During this operation, there is a risk of the product filled in the carton bottles splashing out, to the sides and up. Finally, the finished, filled carton bottles are output from the apparatus 1 via the outfeed station 23. The zones and stations not relevant to the description of the inventive device 3 will not be discussed in detail herein.

In order not to risk recontamination of the carton bottles, or at least the inside thereof, after sterilization agent exposure, it is important to maintain an aseptic environment in an upper part of the apparatus 1 from the venting zone 9 to the sealing zone 13, i.e. until the carton bottles 19 have been finally sealed. This upper part extends somewhere from above the conveyor 17 to the top of the apparatus 1 and is arranged to receive the bottom ends 25 of the carton bottles 19 when these are conveyed through the apparatus 1. This upper part is called an aseptic zone, AZ, and is illustrated in figure 1 (scored area). In the sealing zone 13, the aseptic zone is maintained by means of the device 3 according to the invention, as will be further described below.

It should be said that it, for different reasons, would be very hard to obtain and maintain aseptic conditions throughout the venting, filling and sealing zones. For example, it is difficult control the hygiene of the carrier means 27 and the conveyor 17, inter alia because of hidden surfaces thereof and possible product splashes thereon.

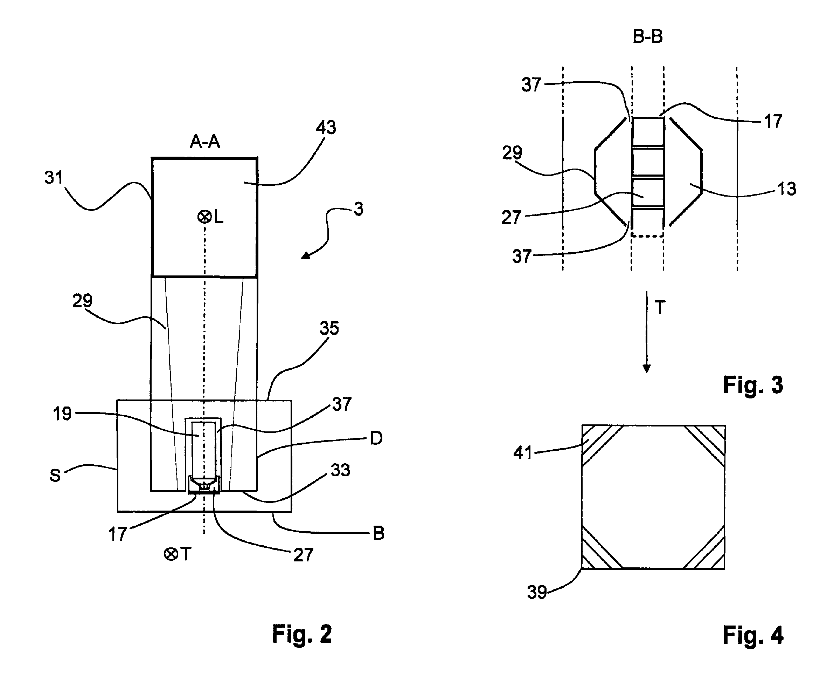

Figure 2 schematically illustrates a cross section of the packaging machine illustrated in figure 1, taken along the line A-A. Figure 3 schematically illustrates a part of a cross section of the packaging machine illustrated in figure 1, taken along the line B-B, which part corresponds to the area of the sealing zone 13. These two figures show the device 3 according to the invention in more detail. The device 3 comprises a duct 29 with a longitudinal center axis L, the duct having a first end 31 arranged to be positioned outside the apparatus 1 and a second end 33 arranged to be positioned inside the apparatus 1, more particularly in the area of the sealing zone 13. The duct 29 extends a predetermined distance into the apparatus 1 through a top wall 35 thereof to partly enclose the sealing zone 13. As apparent from the figures, the duct extension inside the apparatus 1 is such that the duct is arranged on a distance from side S and bottom walls B of the apparatus in order to enable a free way for an air flow out from the duct. This will be further discussed below. Two opposing openings 37 (of which only one can be seen in figure 2) are comprised in the duct 29, extending from the second end 33 and for a certain distance along the longitudinal center axis L thereof, to enable passing of the carton bottles 19 and the carrier means 27 therethrough (the conveyor 17 in this embodiment running immediately below the duct). Thus, in fact, the bottom sealing of the carton bottles 19 take place inside the duct 29. Therefore, the bottom sealing means (not illustrated and not further described herein) are arranged inside the duct above the carton bottles to effect the bottom sealing somewhere at the center, in the transport direction T, of the duct. For the sake of simplicity, all carton bottles, bottom-sealed or not, have been depicted in the same way throughout the figures.

As is apparent from figure 1, the duct is smoothly bent one time approximately 90 degrees (more particularly slightly less than 90 degrees to be drainable) so that the longitudinal center axis L of the duct 29 at the first end 31 is essentially perpendicular to the longitudinal center axis L at the second end 33. Further, a cross section of the duct 29 orthogonal to its longitudinal centre axis L varies along the extension of the duct. At the first end 31 of the duct 29, the cross section is rectangular. At the second end 33 of the duct 29, the cross section is octagonal. More particularly, the cross section is altered from being rectangular to being octagonal and the alteration is made gradually along the complete duct extension. As is illustrated very schematically in figure 4, this is done by cutting the corners 39 of the original rectangle to form a polygon with eight corners, i.e. an octagon, and by continued cutting of edge portions 41 to reduce the size of the octagon to the desired final size.

The device 3 further comprises a HEPA-filter 43 having a rectangular outer shape similar to the duct cross section at the first end 31 of the duct 29. Therefore, the filter 43 fits perfectly at the first end 31 of the the duct, in which position it is easily accessible. Arranged like this, the filter occupies the entire cross section of the duct which makes it impossible for an air flow to travel through the duct 29 without passing the filter 43. Thus, the filter 43 is arranged upstream from the duct bend seen in a direction from the first to the second end of the duct. As previously described, in connection with bottom sealing of the carton bottles, there is a risk of the product filled in the carton bottles splashing out. Since the carton bottle bottom sealing is performed inside the duct 29, the product splashes may hit the inner walls of the duct. However, because of the "hidden" arrangement of the filter beyond the bend of the duct, there is no risk of product splases hitting the filter. This is a huge advantage since the filter is unwashable unlike inner walls of the duct which easily can be cleaned by means of external cleaning nozzles in a fully automated cleaning process.

As previously mentioned, the aseptic zone AZ in the area of the sealing zone 13 is maintained by means of the device 3 according to the invention. This is done by feeding air from outside of the packaging machine through the filter 43, the duct 29 and into the apparatus 1, more particularly the sealing zone 13 thereof. Since the filter 43 is a sterile filter, the air provided to the apparatus will be sterile. The air feeding through the duct 29 is effected by means of a fan (not illustrated) arranged in connection to the duct.

In view of what has been previously described, to maintain the aseptic zone in the area of the sealing zone, the flow of air through the duct 29 should be directed from the first end 31 to the second end 33 of the duct. As was discussed previously, a rectangular cross section of a duct results in circulation zones at the duct corners. Further, a 90 degree bend of a duct with an all through rectangular cross section results in a flow separation at the bend. Both the circulation zones and the flow separation may cause a flow through the duct in a direction opposite to the desired one. In the present context this would involve a risk of unsterile air being drawn from the apparatus and into the duct which would jeopardize the aseptic zone. In turn, this involves a risk of recontamination of the carton bottles. Further, even if use of a bent duct with an all through circular cross section would solve the flow disturbance problems, commercially available filters are rectangular and therefore not suitable for use with such a duct. For economical reasons, use of custom made circular filters is highly non-preferred.

The above flow disturbances will be present to a larger extent in a flow through a bent duct with a rectangular cross section than through a bent duct with a more round cross section. Thus, a flow through a bent duct with a rectangular cross section will be less uniform than a flow through a bent duct with a hexagonal, heptagonal or octagonal cross section. Therefore, by the design of the duct comprised in the device according to the invention, the flow disturbances in at least a part of the duct can be eliminated or at least strongly reduced, because of the octagonal cross section of the duct from the second end 33 and up towards the first end 31. Further, commercially available economically favorable filters can be used in connection with the inventive device because of the rectangular cross section of the duct at the first end 31. Further, by gradually altering the duct cross section as described with reference to figure 4, the cross sectional area in a direction from the first to the second end of the duct will be gradually smaller which will further promote the desired flow direction through the duct, which extends from the first to the second duct end.

The above defined arrangement of the duct 29 in relation to the apparatus 1, which means that the part of the duct extending inside the apparatus 1 is separated from the side S and bottom B walls of the apparatus 1, result in yet another advantage of the present invention. Since there are no obstacles present at the second 33 end of the duct hindering an air flow out of the duct, the risk of that air about to be fed out of the duct changes its flow direction and starts to circulate inside the duct is eliminated or at least strongly reduced. As should be apparent from the above discussion, such a disturbance of the desired one-way flow through the duct could jeopardize the aseptic zone AZ and lead to recontamination of the carton bottles 19.

The above described embodiment should only be seen as an example. A person skilled in the art realizes that this embodiment can be modified and varied in a number of ways without deviating from the inventive conception.

As an example, the purpose of using a bent duct is to protect the filter from product splashing in connection with bottom sealing. For this to be possible, the filter must, of course, be arranged at the right side of the bend, i.e. upstream of the bend seen in the desired flow direction through the pipe. In the above described embodiment, the duct is bent approximately 90 degrees to achieve the above object. However, this object can also be achieved by means of a duct with another design. For example, the duct may be bent less than 90 degrees, even if such a bend could demand a specific arrangement of the filter in relation to the bend to provide filter protection. Further, the duct could comprise more than one bend. However, such alternative designs could make the cleaning of the duct more difficult.

As another example, in the above described embodiment, the inventive device has been used for provision of sterile air to the apparatus. Naturally, the device according to the invention could just as well be used for provision of air with a lower degree of purity, of course in dependence on the specific application.

Further, the duct according to the present invention presents a varying cross section having a "rounder" shape at the second end then at the first end of the duct. In the above described embodiment, the cross section is rectangular at the first end and octagonal at the second end. Obviously, this is just one of many possible variants. For example, the cross section at the second end could instead be decagonal.

Additionally, in the above described device, the duct cross section variation is ongoing along the complete extension of the duct, i.e. the cross section is unique everywhere in the duct. Of course, this is not the only possible way of realizing the invention. The variation could alternatively take place along a part or more parts of the duct, the cross section across the rest of the duct being unaltered.

Further, in accordance with the above described embodiment, the filter is arranged outside the duct to cover the first end thereof. However, the filter could be differently arranged, both inside and outside the duct. As an example, arranged on the outside, the filter may be arranged at a distance from the duct, an interconnection means then being required for connecting the filter with the first end of the duct. In an inside arrangement, the filter may or may not be arranged to extend essentially orthogonal to the longitudinal centre axis of the duct.

Finally, the inventive device may of course be used in connection with packaging machines producing other packages than carton bottles.

It should be stressed that components not necessary for describing the present invention has been omitted, both in the figures and in the text, and that the figures are not drawn according to scale.

## Claims

1. A device (3) for providing a flow of cleaned air to an apparatus (1) comprising a bent duct (29) having a first end (31) arranged to be positioned outside the apparatus and a second end (33) arranged to be positioned inside the apparatus, and a filter (43), air from outside of the apparatus being conveyed through the filter for cleaning, through the duct and into the apparatus, wherein a cross section of the duct orthogonal to a longitudinal centre axis (L) of the same varies, the polygonal cross section at the first end differing from the polygonal cross section at the second end in that the cross section at the second end (33) has more corners than the cross section at the first end (31), wherein that the duct is bent in such a way that a direction of the longitudinal centre axis (L) at the first end (31) is angled in relation to a direction of the longitudinal centre axis at the second end (32), wherein the filter is arranged upstream the bend, relative to the flow of cleaned air, and wherein said cross section is gradually altered along the longitudinal centre axis (L) of the duct (29), from the cross section at the first end (31) to the cross section at the second end (33).

2. A device (3) according to any one of the preceding claims, wherein the filter (43) is arranged to occupy essentially the entire cross section of the duct (29) at a position of the filter.

3. A device (3) according to claim 1, wherein the cross section at the first end (31) is rectangular and the cross section at the second end (33) is octagonal.

4. A device (3) according to any one of the preceding claims, wherein the cross section of the duct (29) at the first end (31) is larger than the cross section of the duct at the second end (33).

5. A device (3) according to any one of the preceding claims, wherein the duct (29) extends a distance into the apparatus (1) to partly enclose a zone (13) where sealing of filled, open packages (19) takes place.

6. A device (3) according to claim 5, wherein the duct (29) extends into the apparatus (1) through a top wall (35) thereof, the duct being arranged separated from side and bottom walls (S, B) of the apparatus to enable a free way for the flow of air out from the duct.

7. A device (3) according to any one of claims 5-6, wherein the duct (29), at the second end (33), comprises two opposing openings (37) extending along the longitudinal center axis (L) thereof, to enable passing of the packages through the duct.

## Patentansprüche

1. Vorrichtung (3) zur Bereitstellung einer Strömung gereinigter Luft an eine Einrichtung (1), die Folgendes umfasst:
einen gebogenen Kanal (29), der ein erstes Ende (31), das zum Positionieren außerhalb der Vorrichtung angeordnet ist, und ein zweites Ende, das zum Positionieren in der Einrichtung angeordnet ist, aufweist, und
einen Filter (43), wobei Luft von außerhalb der Einrichtung durch den Filter zur Reinigung durch den Kanal und in die Einrichtung befördert wird,
wobei ein Querschnitt des Kanals orthogonal zu einer Längsmittenachse (L) davon variiert,
wobei der Querschnitt am ersten Ende anders ist als der polygonale Querschnitt am zweiten Ende und der Querschnitt am zweiten Ende (33) mehr Ecken als der Querschnitt am ersten Ende (31) aufweist,
wobei der Kanal derart gebogen ist, sodass eine Richtung der Längsmittenachse (L) am ersten Ende (31) in Bezug auf eine Richtung der Längsmittenachse am zweiten Ende (32) in einem Winkel angeordnet ist,
wobei der Filter stromaufwärts der Biegung in Bezug auf die Strömung gereinigter Luft angeordnet ist, und
wobei der Querschnitt entlang der Längsmittenachse (L) des Kanals (29) vom Querschnitt am ersten Ende (31) zum Querschnitt am zweiten Ende (33) stufenweise verändert wird.

2. Vorrichtung (3) nach Anspruch 1, wobei der Filter (43) zum Einnehmen des im Wesentlichen gesamten Querschnitts des Kanals (29) an einer Filterposition angeordnet ist.

3. Vorrichtung (3) nach Anspruch 1, wobei der Querschnitt am ersten Ende (31) rechteckig ist und der Querschnitt am zweiten Ende (33) oktagonal ist.

4. Vorrichtung (3) nach einem der vorherigen Ansprüche, wobei der Querschnitt des Kanals (29) am ersten Ende (31) größer ist als der Querschnitt des Kanals am zweiten Ende (33).

5. Vorrichtung (3) nach einem der vorherigen Ansprüche, wobei sich der Kanal (29) über einen Abstand in die Einrichtung (1) erstreckt, um teilweise eine Zone (13) zu umschließen, in der das Abdichten gefüllter, offener Verpackungen (19) ausgeführt wird.

6. Vorrichtung (3) nach Anspruch 5, wobei sich der Kanal (29) in die Einrichtung (1) durch eine obere Wand (35) davon erstreckt, wobei der Kanal getrennt von den Seiten- und Bodenwänden (S, B) der Vorrichtung angeordnet ist, um einen freien Weg für die Luftströmung aus dem Kanal zuzulassen.

7. Vorrichtung (3) nach einem der Ansprüche 5 bis 6, wobei der Kanal (29) am zweiten Ende (33) zwei gegenüberliegende Öffnungen (37) aufweist, die sich entlang der Längsmittenachse (L) davon erstrecken, um das Durchlaufen von Verpackungen durch den Kanal zuzulassen.

## Revendications

1. Dispositif (3) pour fournir un flux d'air nettoyé à un appareil (1), comprenant un conduit coudé (29) ayant une première extrémité (31) agencée de manière à être positionnée à l'extérieur de l"appareil et une deuxième extrémité (33) agencée de manière à être positionnée à l'intérieur de l'appareil, et un filtre (43), de l'air provenant de l'extérieur de l'appareil étant transporté à travers le filtre en vue d'un nettoyage, à travers le conduit et à l'intérieur de l'appareil, une section transversale du conduit orthogonale à un axe central longitudinal (L) de celui-ci variant, la section transversale polygonale à la première extrémité différant de la section transversale polygonale à la deuxième extrémité en ce que la section transversale à la deuxième extrémité (33) a plus de coins que la section transversale à la première extrémité (31), le conduit étant coudé de telle sorte qu'une direction de l'axe central longitudinal (L) à la première extrémité (31) soit inclinée par rapport à une direction de l'axe central longitudinal à la deuxième extrémité (32), le filtre étant agencé en amont du coude, par rapport au flux d'air nettoyé, et ladite section transversale étant graduellement modifiée le long de l'axe central longitudinal (L) du conduit (29), à partir de la section transversale à la première extrémité (31) jusqu'à la section transversale à la deuxième extrémité (33).

2. Dispositif (3) selon la revendication 1, dans lequel le filtre (43) est agencé de manière à occuper essentiellement toute la section transversale du conduit (29) à une position du filtre.

3. Dispositif (3) selon la revendication 1, dans lequel la section transversale à la première extrémité (31) est rectangulaire et la section transversale à la deuxième extrémité (33) est octogonale.

4. Dispositif (3) selon l'une quelconque des revendications précédentes, dans lequel la section transversale du conduit (29) à la première extrémité (31) est plus grande que la section transversale du conduit à la deuxième extrémité (33).

5. Dispositif (3) selon l'une quelconque des revendications précédentes, dans lequel le conduit (29) s'étend sur une distance à l'intérieur de l'appareil (1) de manière à renfermer partiellement une zone (13) où a lieu le scellement d'emballages (19) ouverts remplis.

6. Dispositif (3) selon la revendication 5, dans lequel le conduit (29) s'étend à l'intérieur de l'appareil (1) à travers une paroi supérieure (35) de celui-ci, le conduit étant agencé de manière séparée de parois latérales et inférieure (S, B) de l'appareil de manière à autoriser une voie libre pour le flux d'air hors du conduit.

7. Dispositif (3) selon l'une quelconque des revendications 5 et 6, dans lequel le conduit (29) comprend, à la deuxième extrémité (33), deux ouvertures opposées (37) s'étendant le long de l'axe central longitudinal (L) de celui-ci, pour autoriser le passage des emballages à travers le conduit.
